(19) Europäisches Patentamt
European Patent Office
Office européen des brevets

(11) Veröffentlichungsnummer: **0 120 019**
**B1**

# (12) EUROPÄISCHE PATENTSCHRIFT

(45) Veröffentlichungstag der Patentschrift: **17.12.86**

(51) Int. Cl.⁴: **A 61 K 31/48**

(21) Anmeldenummer: **83901364.6**

(22) Anmeldetag: **29.04.83**

(86) Internationale Anmeldenummer: **PCT/DE 83/00078**

(87) Internationale Veröffentlichungsnummer: **WO 83/03758 (10.11.83 Gazette 83/26)**

## (54) PHARMAZEUTISCHE ZUBEREITUNGEN MIT ZYTOSTATISCHER WIRKUNG.

(30) Priorität: **03.05.82 DE 3216870**

(43) Veröffentlichungstag der Anmeldung: **03.10.84 Patentblatt 84/40**

(45) Bekanntmachung des Hinweises auf die Patenterteilung: **17.12.86 Patentblatt 86/51**

(84) Benannte Vertragsstaaten: **AT BE CH DE FR GB LI LU NL SE**

(56) Entgegenhaltungen:
**EP-A-0 021 206**
**EP-A-0 026 671**
**EP-A-0 032 684**
**EP-A-0 056 358**
**CH-A-367 762**
**DE-A-1 926 045**
**DE-A-2 238 540**
**DE-A-2 524 575**
**DE-A-2 656 344**
**US-A-3 681 497**
**US-A-4 054 660**

**Collection of Czechoslovak Chemical Communications, Band 42, Nr. 4, 1977, (Prag, CZ), M. Beran et al.: "Some N-(D-6-methyl-8-ergolin-I-ylmethyl)-N'-substituted ureas and N-(D-6-methyl-2-chloro-8-isoergolin--yl)-N',N'-diethyl urea", Seite 1417-20**

(73) Patentinhaber: **SCHERING AKTIENGESELLSCHAFT**
**Berlin und Bergkamen, Müllerstrasse 170/178**
**Postfach 65 03 11, D-1000 Berlin 65 (DE)**

(72) Erfinder: **BIRKMAYER, Jörg, Schwarzspanierstrasse 16, A-1090 Wien (AT)**
Erfinder: **HOROWSKI, Reinhard, Am Rosenanger 32, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 120 019 B1

LIBER, STOCKHOLM 1986

**Beschreibung**

Es ist seit langem bekannt, daß Ergot-Alkaloide pharmakologisch wirksam sind. In neuerer Zeit sind therapeutsche Anwendungen einiger Vertreter bekannt geworden. Zum Beispiel wird das Bromocriptin (DE AS 1 926 045) als Laktationshemmer in der Therapie angewendet. Lisuridhydrogenmaleat (US PS 3,681,497) ist als Migränemittel bekannt. Trans-dihydro-Lisurid (DE AS 2.238.540) wirkt pharmakologisch, z.B. als Nidationshemmer.

Aus US 4,054,660 sind Ergoline mit Antitumorwirkung bei Dimethylbeuzanthoacen -induzierten Tumosen in Ratten bekannt. Außerdem eignen sich diese Ergoline zur Behandlung von Prolactin-abhängigen Tumoren. Im DE-OS 252 475 werten weitere Ergolinderivate mit Antitumorwirkung bei Prolactin-ablängigen Tumoren beschrieben.

Es wurde nun gefunden, daß die erfindungsgemäß verwendeten Ergoline Lisurid, Tergurid, Protegurid und Bromergurid zytostatisch wirken.

Sie hemmen in vitro die Vermehrung (Proliferation) von menschlichen Leukämiezellen und Lungenkarzinomzellen.

Hierzu wurden folgende Untersuchungen angestellt:

Menschliche Leukämiezellen und Lungenkarzinomzellen wurden in vitro mit einem Kulturmedium (Minimal essentielles Medium mit 10 % fötalem Kälberserum) gezüchtet, dem steigende Konzentrationen von Lisurid (5, 20, 40 und 100 µg/ml) in Form des Hydrogenmaleats zugesetzt wurden und das jeden 2. Tag durch frisches Medium mit Lisurid ersetzt wurde, wobei sich die Menge des Ergot-Alkaloids auf die freie Base Lisurid bezieht. Die Wachstumskinetik, d.h. die Vermehrungsrate pro Zeiteinheit, wurde durch Bestimmung der Zellzahl nach 48, 96 und 144 Stunden ermittelt und mit der von Zellen verglichen, die in Lisurid-freiem Medium kultiviert wurden.

Es konnte eine Dosis- und Zeit-abhängige Hemmung der Zellvermehrung beobachtet werden, deren Maximalwerte bei den einzelnen Konzentrationen im folgenden aufgeführt sind:

5 µg/ml bis 18 % Hemmung
20 µg/ml bis 53 % Hemmung
40 µg/ml bis 68 % Hemmung
100 µg/ml mehr als 100 % Hemmung.

Bei einer Konzentration von 100 µg/ml zeigte Lisurid neben einer zytostatischen somit auch eine zytotoxische Wirkung. Unter gleichen Versuchsbedingungen geprüfte Normalzellen, wie adulte und embryonale Lungenfibroblasten wurden selbst von hohen Lisurid-Dosen, wie 100 µg/ml, wenn überhaupt nur minimal gehemmt.

Des weiteren wurde in vivo der gleiche Befund an Kleinnagern bestätigt.

Hierzu wurden Han-Wistar-Ratten während 104 - 107 Wochen mit 0,02, 0,2 und 1,0 mg Lisurid/kg oral behandelt. Dabei ließ sich die - beim Nager überwiegend tumorbedingte- Sterblichkeit dosisabhängig reduzieren.

Des weiteren wurden NMRI-Mäuse mit 0,02, 0,06 und 0,2 mg Lisurid/kg oral während 104 - 107 Wochen behandelt. Auch hier kam es teilweise zur Verminderung der Mortalität sowie zu einer deutlichen Hemmung des Auftretens von Lungenademokarzinomen und Mäuseleukämien.

Die selektive Wirkung auf Tumore wird auch dadurch unterstützt, daß sich Ergot-Alkaloide wie Lisurid

in einzelnen Organen selektiv anreichern und damit hier zu besonders hoher Wirksamkeit gelangen. Neben Untersuchungen zur Bindung von radioaktivem Lisurid sind hier auch besonders Befunde von Bedeutung, bei welchen sich bei Anwendung von fluoreszenz-optischen Nachweismethoden bei Inkubation tierischer und menschlicher Zellen eine Anreicherung von Lisurid in diesen Zellen zeigte und sich z.B. bei weißen Blutzellen des Menschen eine massive Anreicherung der Lisuridfluoreszenz bei Inkubation dieser Zellen mit $10^{-7}$ - $10^{-5}$-molarer Konzentration von Lisurid fand.

Ergolius hat folgende Struktur:

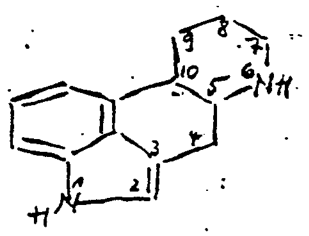

Die erfindungsgemäßen Ergoline haben folgende chemische Beseidumingen:

Lisurid 1,1-Diethyl-3-(6-methyl-9.10-didelhydro-8α-ergolinyl)-harnstoff

Tegurid 1,1-Diethyl-3-(6-methyl-8α-erfolinyl)-harnstoff

Protegurid 1,1-Diethyl-3-(6-u-propyl-8α-ergolinyl)-harnstoff

Bromergürid 1,1-Diethyl-3-(2-brom-6-methyl-9.10-didehydro-8α-ergolinyl)-harnstoff

Die erfindungsgemäß verwendeten Ergoline können in freier Form oder in Form ihrer Additionssalze mit physiologisch verträglichen Säuren, wie Wein-, Malein-, Methansulfon-, Phosphor- und Salzsäure verwendet werden.

Es war durchaus überraschend, daß die anspruchsgemäßen Ergoline, die bekanntermaßen über Monoamin-Receptoren wirken, das Wachstum und die Vermehrung maligner Zellen, wie die von menschlicher Leukämie und Lungenkarzinomen hemmen.

Die erfindungsgemäß verwendeten Ergoline haben den Vorteil gegenüber bekannten Zytostatica, wie Endoxan oder Methotrexat, die auf alle sich teilenden Zellen wachstumshemmend wirken, daß die bei diesen auftretenden erheblichen Nebenwirkungen, wie

Störungen der Blutbildung, im Magen-Darm-Trakt, Haarausfall, der Spermiogenese entfallen.

Dieses wird durch die fehlende wachstumshemmende Wirkung auf normale, nicht-tumorale Zellen in Kultur bestätigt und ebenso dadurch, daß sich bei der Durchführung von Langzeitverträglichkeits- und Karzinogenitätsprüfungen am Tier und auch bei der breiten klinischen Untersuchung am Menschen für die oben angegebenen Substanzen keine dieser Wirkungen zeigen ließen.

Die erfindungsgemäß verwendbaren Wirkstoffe können somit aufgrund dieser fehlenden Nebenwirkungen auch für Therapien eingesetzt werden, die weit über die üblicherweise angewendeten 3 Monate hinausreichen und ebenso bei Patienten, bei welchen die bekannten Zytostatika-Nebenwirkungen wie Abwehrschwäche, Anämie, Blutungsneigung nicht toleriert werden können.

Die erfindungsgemäß verwendeten Ergoline sind zur Behandlung von Leukämie geeignet. Insbesondere sind sie jedoch zur Behandlung von Leukämien und miteinander verwandten Karzinomen wie Bronchial- und Lungenkarzinomen geeignet.

Der Wirkstoff wird in üblicher Form in pharmazeutischen Zubereitungen verabreicht. Beispielsweise kann die pharmazeutische Zubereitung in Form von Tabletten, Pulvern, Granulaten, Kapseln, Sirupen und Elixieren für orale Verabreichung sowie in Form von Lösungen, Suspensionen, Dispersionen und Emulsionen für parenterale Verabreichung, beispielsweise einer sterilen injizierbaren wässrigen Lösung verwendet werden. Die für orale Verabreichung geeigneten Zubereitungen können die üblichen Träger- und Hilfsstoffe enthalten, wobei die Wirkstoffe auch, z.B. für Depotformulierungen, mikroverkapselt sein können.

Die täglich zu verabreichende Menge an Wirkstoff hängt naturgemäß von der Art des zu behandelnden Falles ab. Im allgemeinen werden befriedigende Resultate erreicht mit einem Dosisbereich von etwa 0,01 bis 10 mg pro kg Körpergewicht des Testtieres, die nötigenfalls in 2-3 Anteilen verabreicht werden.

Bei der Anwendung in der Humanmedizin beträgt die zu verabreichende Menge bei oraler Anwendung 0,01 - 50 mg/d, vorzugsweise 0,5 - 10 mg/d. Bei parenteraler Anwendung, z.B. durch Injektion als Intervall- oder durch Infusion als Dauertherapie, beträgt die tägliche Dosis 0,15 - 1,5 mg/d.

Die einzelnen Dosisformen enthalten pro Dosiseinheit 0,01 bis 5 mg Wirkstoff.

Vom Standpunkt der Herstellung und der günstigen Verabreichungsmöglichkeit werden Injektionspräparate in Form von Ampullen oder feste Zubereitungen, insbesondere Kapseln und Tabletten, bevorzugt.

Die nachfolgenden Beispiele dienen zur weiteren Erläuterung der Erfindung.

**Beispiel 1**

Die für parenterale Verabreichung geeigneten Ampullen werden in an sich bekannter Weise hergestellt. Hierzu wird Lisuridhydrogenmaleat (0,0005 g) in Wasser p.i. gelöst, auf 1 ml aufgefüllt und sterilisiert.

**Beispiel 2**

Die für orale Verabreichung geeigneten Tabletten, welche die nachfolgend beschriebenen Bestandsteile enthalten, werden in an sich bekannter Weise hergestellt.

| Bestandteile | Gewicht |
|---|---|
| 1.1-Diethyl-3-(6-methyl-8ß-ergolinyl)-harnstoff-dihydrogen-phosphat | 1,00 mg |
| Laktose | 98,05 mg |
| Maisstärke | 14,00 mg |
| Polyvinylpyrrolidon | 5,00 mg |
| Magnesiumstearat | 0,70 mg |
| Talk | 1,25 mg |
| | 120,00 mg |

**Patentansprüche**

1. Verwendung von Lisurid für die Herstellung eines Arzneimittels zur Behandlung von Leukämie und Lungentumoren.

2. Verwendung von Tergurid für die Herstellung eines Arzneimittels zur Behandlung von Leukämie und Lungentumoren.

3. Verwendung von Protergurid für die Herstellung eines Arzneimittels zur Behandlung von Leukämie und Lungentumoren.

4. Verwendung von Bromergurid für die Herstellung eines Arzneimittels zur Behandlung von Leukamie und Lungentumoren.

5. Verwendung der Verbindungen nach Anspruch 1-4 in Form ihrer Additionssalze mit physiologisch verträglichen Säuren.

6. Verwendung von Lisuridhydrogenmaleat nach Anspruch 1.

7. Verwendung von

Terguriddihydrogenphosphat nach Anspruch 1

8. Verwendung der Verbindungen nach Anspruch 1-7 in einer Menge von 0,01 - 5 mg.

**Claims**

1. Use of lisuride for the preparation of a medicament for the treatment of leukemia and lung tumours.
2. Use of terguride for the preparation of a medicament for the treatment of leukemia and lung tumours.
3. Use of proterguride for the preparation of a medicament for the treatment of leukemia and lung tumours.
4. Use of bromerguride for the preparation of a medicament for the treatment of leukemia and lung tumours.
5. Use of the compounds according to claims 1-4 in the form of their addition salts with physiologically compatible acids.
6. Use of lisuride hydrogen maleate according to claim 1.
7. Use of terguride dihydrogen phosphate according to claim 1.
8. Use of the compounds according to claims 1-7 in an amount of 0.01 - 5 mg.

**Revendications**

1. Application du lisuride pour la préparation d'un médicament destiné au traitement de la leucémie et de tumeurs du poumon.
2. Application du terguride pour la préparation d'un médicament destiné au traitement de la leucémie et de tumeurs du poumon.
3. Application du proterguride pour la préparation d'un médicament destiné au traitement de la leucémie et de tumeurs du poumon.
4. Application du bromerguride pour la préparation d'un médicament destiné au traitement de la leucémie et de tumeurs du poumon.
5. Application de composés selon l'une quelconque des revendications 1 à 4 sous la forme des sels d'addition qu'ils forment avec des acides acceptables du point de vue physiologique.
6. Application de l'hydrogénomaléate de lisuride selon la revendication 1.
7. Application du dihydrogénophosphate de terguride selon la revendication 1.
8. Application de composés selon l'une quelconque des revendications 1 à 7 en une quantité de 0,01 à 5 mg.